# EUROPEAN PATENT APPLICATION

(11) **EP 3 960 217 A1**
(43) Date of publication of application: **02.03.2022**
(21) Application number: 20193237.3
(22) Date of filing: 27.08.2020
(51) Int. Cl.: A61M 1/06

(54) **BREAST PUMP DEVICE AND CONTROLLER FOR THE DEVICE, AND A CONTROL METHOD**

(71) Applicant: Koninklijke Philips N.V., 5656 AG Eindhoven (NL)
(72) Inventor: BOURQUIN, Yannyk Parulian Julian, 5656 AE Eindhoven (NL); BROCKHUIS, Lili-Marjan, 5656 AE Eindhoven (NL); SIMONS, Evelyn Josefina Maria, 5656 AE Eindhoven (NL)
(74) Representative: Philips Intellectual Property & Standards

(57) **Abstract**

A breast pump device is provided, and a controller for operating a pressure source of the breast pump device. A degree of stretching of the nipple is determined during use of the breast pump device. A maximum under-pressure level to be applied during use of the breast pump is determined in dependence on the degree of stretching. This provides automatic pressure level adjustment to provide improved comfort for the user while enabling successful milk expression.

## Description

### FIELD OF THE INVENTION

The invention relates to a breast pump device and a method of operating a breast pump device.

### BACKGROUND OF THE INVENTION

Breast pumps are used by breast feeding women to extract milk from their breast such that the extracted milk can be fed to their babies at a later time.

It is well known that best nutrition for babies is breast milk. The world health organization (WHO) recommends to breast feed babies for at least one year, preferably longer. However, mothers often go back to work after only several weeks or months. To provide the best nutrition to their babies, mothers may then express milk using a breast pump. The expressed milk can be stored and given to the baby at a later stage and/or by somebody else.

For mothers, it is important that using a breast-pump is efficient and comfortable. Nipple pain occurs in up to 96% of breastfeeding mothers, and is one of the main reasons for a mother to stop breastfeeding or stop using a breast pump. Nipple pain tends to occur more often in first-time mothers.

Currently, the user of a breast pump sets the maximum vacuum manually according to their preference. A main issue with this is that the user may not use the correct setting to achieve a maximum milk output without nipple pain. Too often, a too high setting is used, which stretches the nipple too much and ultimately induces nipple pain. Ultimately, the user may stop using the breast pump due to the pain.

It would be beneficial to provide a system which reduces the instances of mothers stopping breastfeeding early due to nipple pain.

### SUMMARY OF THE INVENTION

The invention is defined by the claims.

According to examples in accordance with an aspect of the invention, there is provided a controller for a breast pump device, wherein the controller is for controlling operation of a pressure source of the breast pump device to apply an under-pressure, the controller comprising:
an input for receiving an input signal representing a degree of stretching of the nipple during use of the breast pump device; and
an output for controlling operation of the pressure source thereby to implement a maximum under-pressure level to be applied during use of the breast pump in dependence on the input signal.

This controller operates the pressure source of a breast pump device so that during milk expression a maximum under-pressure level is set, which depends on the degree of stretching experienced by the particular user of the system. In this way, the maximum under-pressure level may be set automatically in such a way as to reduce or minimize discomfort to the user. The "maximum under-pressure level" is the maximum amount by which the pressure is below atmospheric pressure, and hence the greatest level of vacuum, corresponding to a minimum absolute pressure level.

The controller for example implements a cyclic control waveform for the pressure source during milk expression, and the maximum under-pressure level implemented by this control waveform is adapted based on the input signal.

The controller is for example adapted to derive a relationship between the level of the under-pressure and the degree of stretching of the nipple, and to set the maximum under-pressure level based on the relationship.

This relationship depends on the individual user, so the controller implements customized pressure level setting.

The controller is for example adapted to derive the relationship during a calibration routine. In this way, the relationship is established already when the use of the breast pump device for actual milk expression is started. It is instead possible for the controller to learn the relationship during normal use of the breast pump device.

The calibration routine for example comprises the application of a ramp under-pressure waveform. This enables the degree of stretching for all under-pressure levels to be established.

The relationship may have a maximum degree of stretching and the maximum under-pressure level is selected as the pressure level corresponding to a predetermined fraction of the maximum degree of stretching. In this way, the maximum under-pressure level is set such that the nipple is only exposed to a desired fraction (e.g. 90%) of the maximum possible stretching, for that particular user.

The controller is for example adapted to adjust the maximum under-pressure level during a breast pumping session. The maximum under-pressure level may be set as a constant for a full breast pumping session, but in this example the maximum under-pressure level may vary over time. This may enable the controller to take account of the different relationship between stretching and under-pressure level for different levels of breast fullness. This results from different breast elasticity for different breast fullness.

The controller is for example adapted to set the maximum under-pressure level further in dependence on additional parameters comprising one of more of:
weather conditions;
temperature conditions of the breast.

Thus, additional parameters may be taken into account, which either influence the actual stretching or influence the pain felt in response to nipple stretching.

A most basic implementation of the maximum under-pressure level is a capping function without changing the duration of each cycle. The controller may instead be adapted to control a frequency of a cyclic under-pressure waveform in dependence on the maximum under-pressure. By reducing the maximum under-pressure, the cycle may become shorter, and hence a higher frequency may be applied. In this way, long periods of capped constant under-pressure level are avoided.

The invention also provides a breast pump device, comprising
at least one breast receiving portion configured to receive a breast of a user;
a pressure source coupled to the at least one breast receiving portion and being configured to generate at least an under-pressure;
a sensor for generating said input signal representing a degree of stretching of the nipple during use of the breast pump device; and
the controller as defined above.

This provides the application of the controller to a breast pump device.

A user input interface may be provided for receiving user feedback. The user feedback for example enables a user to input their level of discomfort, or else to manually adjust (i.e. override or re-set) the maximum under-pressure level that has been set by the system. The controller may thus be adapted to set the maximum under-pressure level further in dependence on the user feedback.

The sensor may comprise:
an optical sensor;
a camera and image processor;
a distance measurement system for a measuring distance to the nipple of the breast of the user; or
a volume determining system for determining an occupied volume of the breast receiving portion.

Various sensors are possible for measuring (directly or indirectly) the nipple stretch.

The invention also provides a computer-implemented method of controlling a breast pump device, comprising:
receiving an input signal representing a degree of stretching of the nipple during use of the breast pump device; and
controlling operation of the pressure source of the breast pump device, thereby to implement a maximum under-pressure level to be applied during use of the breast pump in dependence on the input signal.

The method may comprise deriving a relationship between the level of the under-pressure and the degree of stretching of the nipple, and setting the maximum under-pressure level based on the relationship.

The method may be implemented in software.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows the typical parts of a known breast pump system;
Figure 2 shows one example of breast pump design in more detail;
Figure 3 shows a most basic sensor example in which a nipple receiving area (neck) of the breast receiving funnel is provided with an optical sensor arrangement;
Figure 4 shows an example of the relationship between nipple stretching (y-axis) and vacuum level;
Figure 5 shows an example of the pressure cycle during expression applied by the pump unit of the operating unit;
Figure 6 shows a first way to implement the maximum under-pressure level; and
Figure 7 shows a second way to implement the maximum under-pressure level.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a breast pump device and a controller for operating a pressure source of the breast pump device. A degree of stretching of the nipple is determined during use of the breast pump device. A maximum under-pressure level to be applied during use of the breast pump is determined in dependence on the degree of stretching. This provides automatic pressure level adjustment to provide improved comfort for the user while enabling successful milk expression.

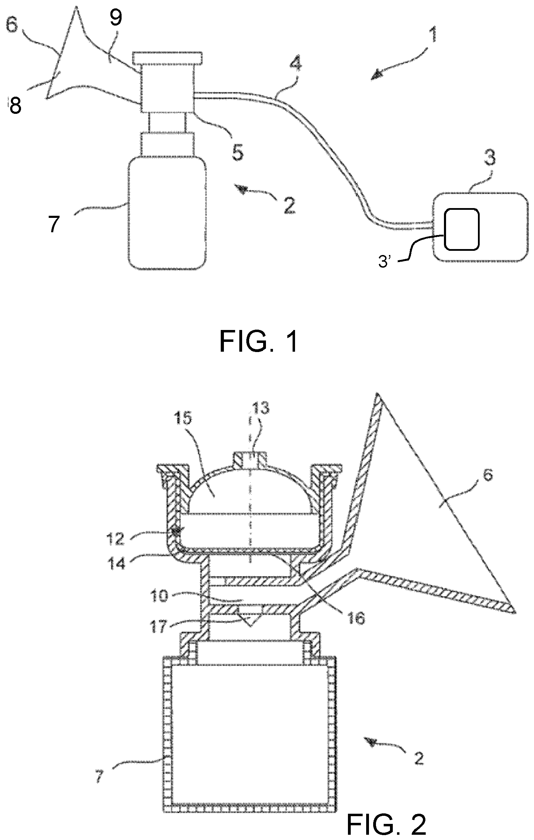

Figure 1 shows the typical parts of a known breast pump system. The breast pump system 1 comprises a breast pump 2, also known as an expression unit, and an operating unit 3. The operating unit is basically a pump unit (vacuum pump), associated hardware, and a pump controller 3'.

The breast pump 2 and the operating unit 3 are connected by a hose 4. The hose 4 provides a fluid communication between the breast pump 2 and the operating unit 3. The hose 4 may also be used to provide an electrical connection between the breast pump 2 and the operating unit 3. For example, the hose 4 may supply an operating signal or electrical power between the breast pump and the operating unit. In an alternative embodiment, the operating unit 3 is directly mounted and connected to the breast pump 2.

The breast pump 2 has a main body 5, a funnel 6 and a collection vessel 7. The collection vessel 7 collects milk expressed from a user's breast and may take the form of a feeding bottle or bag or any suitable container. The collection vessel 7 is attached to the main body 5 by a screw fitting, although it will be understood that alternative releasable attachment means may be used, such as clips (not shown).

The breast-receiving funnel 6 extends from the main body 5. The funnel 6 is configured to receive the breast of a user. The funnel 6 has a mouth 8 and a throat 9. The mouth 8 is open at an outer end of the funnel 6 to receive a user's breast, and the funnel 6 converges from the outer end towards the throat 9 to form a hollow recess in which a breast is received.

The main body 5 fluidly connects the funnel 6 to the collection vessel 7. A fluid passageway 10 (shown in Figure 2) is formed through the main body 5 from the breast receiving space of the funnel 6 to the collection vessel 7. The main body 5 is formed from an outer shell. The main body 5 is integrally formed with the funnel 6, however it will be understood that the funnel 6 may be detachable. In the present arrangement, the main body 5 is formed from polypropylene, although it will be understood that alternative suitable materials may be used.

Referring now to Figure 2, a chamber 12 is formed in the main body 5. The chamber forms part of a vacuum path. The chamber 12 is in fluid communication with the fluid passageway 10 between the funnel 6 and the collection vessel 7. The chamber 12 has a vacuum port 13. The vacuum port 13 provides a port to communicate with the operating unit 3. The hose 4 is mountable to the vacuum port 13 to fluidly connect the chamber 12 with the operating unit 3.

A membrane 14 is received in the chamber 12. The membrane 14, also known as a diaphragm, is flexible. An outer rim of the membrane 14 is mounted to the chamber 12. The membrane 14 separates the chamber 12 into a first space 15 and a second space 16. The first space 15 communicates with the vacuum port 13. The first space 15 forms part of a first section of the vacuum path. The second space 16 communicates with the fluid passageway 10 between the breast receiving space of the funnel 6 and the collection vessel 7. The second space 16 forms part of a first section of the vacuum path.

A one-way valve 17 is disposed in the fluid passageway 10. The one-way valve 17 prevents a pressure reduction being formed in the collection vessel 7. The one-way valve is for example a duckbill valve.

The membrane 14 is for example formed from silicone. However, it will be understood that the membrane 14 may be formed from another suitable material.

The flexible membrane 14 has a predefined shape. In the present arrangement, the membrane 14 has a substantially cup-shaped arrangement in a neutral condition. That is, when the membrane 14 is received in the chamber 12, but has not been deformed. However, it will be understood that the membrane 14 may have an alternative shape.

The operating unit 3 comprises a pump controller 3', a power source, a motor and a pump unit actuated by the motor. The pump unit is configured to generate and release a pressure reduction (i.e. vacuum) in the vacuum path, for example using a pressure relief valve separate to the pump unit, although these may be combined into a single unit. The controller 3' controls operation of these components of the operating unit 3.

The controller 3' operates the pump unit with sequence of strokes, each stroke comprising the vacuum generation phase and the vacuum release phase. During vacuum generation, the breast is stimulated to express milk. This milk flows to the fluid passageway 10. During the vacuum release phase, the milk passes through the one-way valve 17 into the collection vessel. A small opening is for example provided to allow air to escape from the bottle. This may be located at the screw connection.

The invention provides a system for measuring a degree of stretching of the nipple and uses this to alter the vacuum cycle of the breast pump, in particular by setting a maximum under-pressure level to be applied during use of the breast pump.

The degree of nipple stretching may be determined in various ways.

Several examples are for example explained in WO 2019/145163, in which the amount of nipple elongation is used as a measure of breast fullness. Examples of ways to measure nipple elongation include:
a camera for capturing images of the nipple, with nipple elongation determined by image analysis. The camera could be directly implemented in the breast pump or could be an external camera, such as from a smartphone;
a distance sensor arranged opposite a tip of the nipple aligned axially with the direction in which the nipple elongates. This may be based on an ultrasound sensor, using time of flight measurement;
a pressure estimation unit for estimating a drop in the under-pressure level; within the breast receiving funnel. This involves a surrogate estimation of the nipple elongation from the drop of under-pressure within the vacuum chamber that is dependent on the volume of the chamber. As the nipple elongates, the volume of the chamber decreases so the under-pressure level decreases; and
a nipple shape measurement unit. Some designs of breast-receiving funnel include a collapsible membrane. By placing a shape sensor or accelerometer on the collapsible part of the funnel, the change in shape of the membrane can be measured, and this shape varies in dependence on the degree of nipple stretching.

Thus, there are many suitable known ways to measure the nipple elongation. Any of these known approaches may be employed.

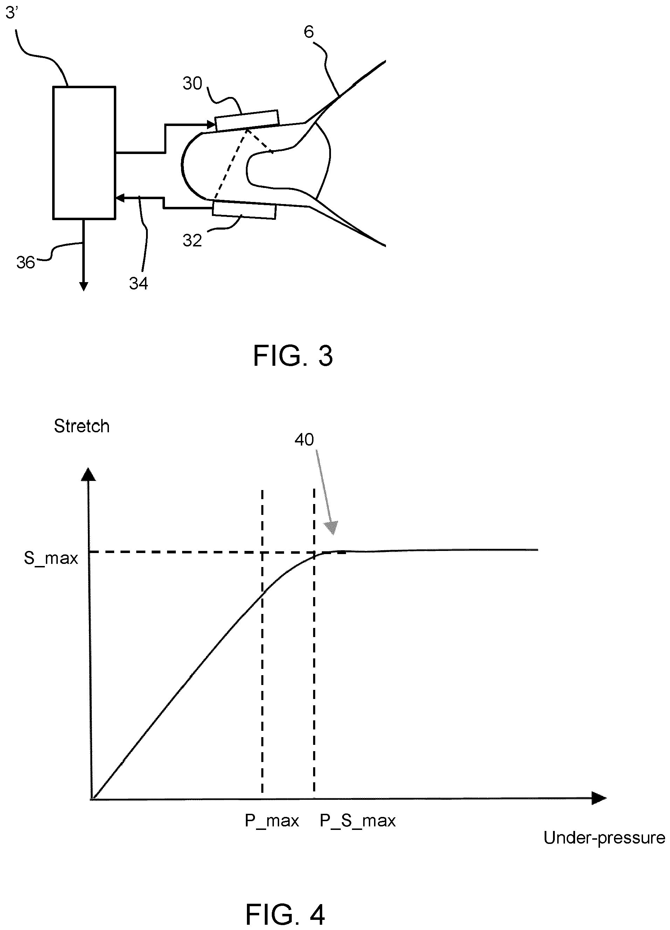

Figure 3 shows a most basic example in which a nipple receiving area (neck) of the breast receiving funnel 6 is provided with an optical sensor arrangement, comprising an array 30 of LEDs and an optical sensor or optical sensor array 32 (comprising photodiodes) at the opposite side of the breast-receiving funnel 6. The optical sensor arrangement 30,32 generates an input signal 34 to the controller 3' representing the amount of nipple stretching and the controller 3' has an output 36 for controlling the pump unit of the operating unit 3.

The light from the array 30 of LEDs is highly divergent, and propagates within the breast shield, for detection by the optical sensor arrangement. When the nipple is placed within the funnel and stretched due to the vacuum, the presence of the nipple scatters the light. The detection signal can then be analyzed to determine the amount of nipple stretching, correlated with the amount of light scattering.

An alternative optical solution is simply multiple line of sight sensors (each comprising an LED with a single output direction towards an associated detector). The boundary between interrupted and non-interrupted lines of sight indicates the forward extent reached by the nipple and hence the degree of elongation.

To avoid effects due to ambient light, infrared light may be used in any optical solution. Additionally, pulsed light can be used to remove any effect of ambient light. The light may be pulsed at a specific frequency, and the signal analysis at the detector may then be based on analysis of the amplitude of the signal with the specific frequency component.

Regardless of the way in which the nipple stretching is measured, the relationship between the amount of stretching data and the level of under-pressure applied to the breast-receiving funnel (i.e. the vacuum level) is derived by the controller 3'. The relationship can be obtained from several pumping cycles. The invention is based on then setting the maximum under-pressure level based on the relationship. In this way, the degree of discomfort may be reduced while still providing sufficient vacuum for successful breast pumping.

The relationship may be obtained during a calibration routine, for example involving the application of a slowly ramped under-pressure waveform from atmospheric pressure to a maximum under-pressure level.

Figure 4 shows an example of the relationship between nipple stretching (y-axis) and vacuum level (i.e. the under-pressure amount by which the pressure is below atmospheric pressure, x-axis).

The relationship has a maximum degree of stretching S_max reached at the elbow 40 of the curve. The vacuum level P_S_max at this point is user-specific.

A maximum under-pressure level P_max may then be selected as the under-pressure level corresponding to a predetermined fraction of the maximum degree of stretching (and hence, since there is a one-to-one relationship, equivalently this will be at a predetermined fraction of the under-pressure level).

The maximum under-pressure P_max may for example be an under-pressure level at 90% of the under-pressure level corresponding to the maximum stretch at elbow 40. Of course, other models could be used such as using the slope of the curve, etc.

The maximum under-pressure may also be adjusted by taking into account input from the user. The user can input in a system (smartphone or directly to the breast pump system) information such as the state of the nipple and the sensation i.e. amount of discomfort during expression. If the user feels that the expression session caused too much nipple pain, the system could then lower the threshold used to set the maximum under-pressure level, e.g. from 90% to 80%.

By reducing the maximum applied under-pressure so that healing can take place and future breast pumping can be less painful, the risk of the mother stopping breast feeding or pumping completely is reduced. Feedback can be given so that the mother is aware that future breast pumping sessions should be less painful.

It is known that during milk expression, the extent of nipple stretching increases as the breast empties. The maximum under-pressure may be set at the start of the session with a constant maximum under-pressure to be used throughout the breast pumping session. However, to take account of the different nipple stretch function at different states of fullness, the maximum under-pressure may be adapted dynamically, based on the measured nipple stretching and based on a stretching model of the breast over time, or based on previously recorded data for the same user.

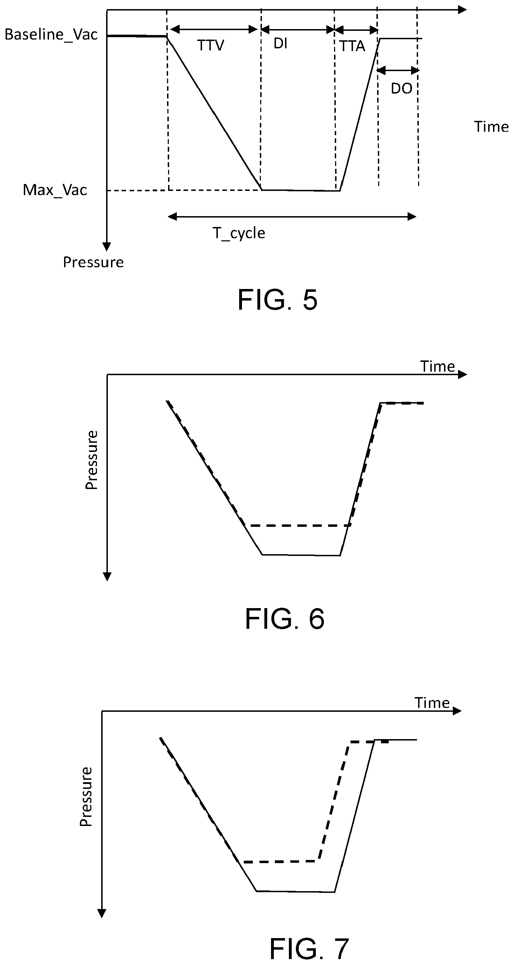

Figure 5 shows an example of the pressure cycle during expression applied by the pump unit of the operating unit 3.

The control signal to the pump unit, and hence the pressure, cycles between a baseline vacuum "Baseline_Vac" and a maximum vacuum (i.e. a maximum under-pressure) "Max_Vac".

There is first a time to (maximum) vacuum, TTV, then a dwell-in time DI, then a time to atmosphere (or to the baseline vacuum), TTA, and finally a dwell-out time, DO. The vacuum rate is defined as Max_Vac/TTV and the atmospheric rate is defined as Max_Vac/TTA.

By way of example:
Baseline_Vac = atmospheric pressure
Max Vac = 280 mbar (28kPa) below atmospheric pressure
TTV = 0.85 s
DI = 0.3 s
TTA = 0.05 s
DO = 0.35 s

This gives a total cycle time T_cycle of 1.55s.

Figure 6 shows a first way to implement the maximum under-pressure level. This is simply a capping function so that the dwell-in time DI is lengthened whereas the TTV and TTA periods are shortened. The total cycle time T_cycle remains the same.

Figure 7 shows a second way to implement the maximum under-pressure level. The under-pressure is capped, but the dwell-in time is kept the same. Thus, the TTA is earlier, and the total cycle time is shortened. The cyclic frequency is correspondingly increased.

These are just two example of how to implement the maximum under-pressure level.

It is noted that other conditions such as weather and temperature, temperature of the nipple (for example if there is preheating prior to use of the breast pump) are factors that may change not only the nipple stretching but also the sensation of nipple stretching. Thus, the stretching model could also include this type of information either using input from the user or from external sources, such as weather station data or home monitoring systems. A sensor may also be used in the breast pump, e.g. a thermal sensor in the breast shield, which would measure ambient temperature before use and skin temperature during use.

The user could also provide direct feedback.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A controller (3') for a breast pump device, wherein the controller is for controlling operation of a pressure source (3) of the breast pump device to apply an under-pressure, the controller comprising:
an input (34) for receiving an input signal representing a degree of stretching of the nipple during use of the breast pump device; and
an output (36) for controlling operation of the pressure source thereby to implement a maximum under-pressure level (P_max) to be applied during use of the breast pump in dependence on the input signal.

2. The controller of claim 1, adapted to derive a relationship between the level of the under-pressure and the degree of stretching of the nipple, and to set the maximum under-pressure level based on the relationship.

3. The controller of claim 2, adapted to derive the relationship during a calibration routine.

4. The controller of claim 3, wherein the calibration routine comprises the application of a ramp under-pressure waveform.

5. The controller of any one of claims 2 to 4, wherein the relationship has a maximum degree of stretching (S_max) and the maximum under-pressure level (P_max) is selected as the under-pressure level corresponding to a predetermined fraction of the maximum degree of stretching.

6. The controller of any one of claims 1 to 5, adapted to adjust the maximum under-pressure level (P_max) during a breast pumping session.

7. The controller of any one of claims 1 to 6, adapted to set the maximum under-pressure level further in dependence on additional parameters comprising one of more of:
weather conditions;
temperature conditions of the breast.

8. The controller of any one of claims 1 to 7, adapted to control a frequency of a cyclic under-pressure waveform in dependence on the maximum under-pressure.

9. A breast pump device, comprising
at least one breast receiving portion (6) configured to receive a breast of a user;
a pressure source (3) coupled to the at least one breast receiving portion and being configured to generate at least an under-pressure;
a sensor (30,32) for generating said input signal representing a degree of stretching of the nipple during use of the breast pump device; and
the controller of any one of claims 1 to 8.

10. The device of claim 9, further comprising a user input interface for receiving user feedback.

11. The device of claim 10, wherein the controller is adapted to set the maximum under-pressure level further in dependence on the user feedback.

12. The device of any one of claims 9 to 11, wherein the sensor comprises:
an optical sensor (30,32);
a camera and image processor;
a distance measurement system for a measuring distance to the nipple of the breast of the user; or
a volume determining system for determining an occupied volume of the breast receiving portion.

13. A computer-implemented method of controlling a breast pump device, comprising:
receiving an input signal representing a degree of stretching of the nipple during use of the breast pump device; and
controlling operation of the pressure source of the breast pump device, thereby to implement a maximum under-pressure level to be applied during use of the breast pump in dependence on the input signal.

14. The method of claim 13, comprising deriving a relationship between the level of the under-pressure and the degree of stretching of the nipple, and setting the maximum under-pressure level based on the relationship.

15. A computer program comprising computer program code which is adapted, when said program is run on a computer, to implement the method of claim 14.
